# EUROPEAN PATENT SPECIFICATION

(11) **EP 2 473 611 B1**
(45) Date of publication and mention of the grant of the patent: **07.12.2016**
(21) Application number: 10814024.5
(22) Date of filing: 03.09.2010
(51) Int. Cl.: C12P 5/02, A01K 61/00, A01K 67/033, C05F 1/00, C05F 17/00

(54) **A SYSTEM TO PRODUCE FEEDSTOCK FOR BIOGAS PRODUCTION**
SYSTEM ZUR HERSTELLUNG EINES ROHSTOFFS ZUR BIOGASHERSTELLUNG
SYSTÈME POUR PRODUIRE UNE CHARGE D'ALIMENTATION POUR UNE PRODUCTION DE BIOGAZ

(30) Priority: 04.09.2009 SE 0901154
(43) Date of publication of application: 11.07.2012
(73) Proprietor: Norén, Fredrik, 45330 Lysekil (SE)
(72) Inventor: Norén, Fredrik, 45330 Lysekil (SE)
(74) Representative: Copsey, Timothy Graham
(86) International application number: PCT/SE2010/000215
(87) International publication number: WO 2011/028163

(56) References cited:
- JP-A- H0 857 462
- JP-A- 2000 033 358
- JP-A- 2008 049 273
- US-A- 4 975 106
- US-A1- 2003 111 020
- LIM Y.-G. ET AL: 'Solubilization and methanogenesis of blue mussels in saline mesophilic anaerobic biodegradation' INTERNATIONAL BIODETERIORATION AND BIODEGRADATION vol. 61, no. 3, 2007, pages 251 - 260, XP022537666
- OLOFSSON E. ET AL: 'Odling av Blamusslor fran flotte' FISKERIVERKET. DNR-231-0908-04 31 May 2008, pages 1 - 6, XP003027924
- KANG K.H. ET AL: 'Influence of water temperature on spawning induction and larval development of the sea squirt Halocynthia ritteri (Oka, 1906)' AQUACULTURE RESEARCH vol. 40, no. 5, March 2009, pages 513 - 518, XP003027925
- KELLY M.S. ET AL: 'Marine Estate Research Report - The potential of marine biomass for anaerobic biogas production', 2008 XP003027926
- VERGARA-FERNANDEZ A. ET AL: 'Evaluation of marine algae as a source of biogas in a two-stage anaerobic reactor system' BIOMASS AND BIOENERGY vol. 32, no. 4, April 2008, pages 338 - 344, XP022546877
- CHAN G.Y.S. ET AL: 'Effects of leachate recirculation on biogas production from landfill co-disposal of municipal solid waste, sewage sludge and marine sediment' ENVIRONMENTAL POLLUTION vol. 118, no. 3, 2002, pages 393 - 399, XP003027927

## Description

### BACKGROUND

In industrial production of biogas the availability of feedstock is limited. Biogas is defined in literature as a gas mixture mainly consisting of methane (CH₄) and carbon dioxide (CO₂), which is produced by bacterial breakdown of a biological substrate. Industrial biogas plants are forced to buy biomass, e.g. ley and wheat, from farmers to produce biogas. This means that the product, biogas, can be uneconomic to produce. The area of arable land is also limited since there is a competition situation between crops for food and for energy production. The loss in production is also strongly weather dependent, since bad weather is decreasing the crops. These factors lead to that the product, biogas, may become uneconomical to produce.

Earlier ideas, about alternative marine biomass for anaerobic digestion, have dealt with microalgae, macroalgae or blue mussels. All these have drawbacks as feedstock for production of biogas; Microalgae have a high cost of culturing considering that specific nutrient must be added under sterile conditions as well as a need for extra illumination since day light is insufficient. Macroalgae has been shown to be a good feedstock for anaerobic digestion but can, if they are harvested from beaches, contain high concentrations of heavy metals such as cadmium. By culturing macroalgae offshore it is possible to improve the economical prerequisites by fertilizing the water and hence enhance the growth rate. Blue mussels have a large draw back in that they have non-organic shells which a biogas plant does not want to include in the process since the shells clogs process pipes as well as increase the sedimentation of not digestible matter.

Producers of biogas always strive to increase the production of gas given a certain amount of biomass to maximize the yield. The problem to be solved is how the producer can increase the gas production. The most beneficial solution of biomass production is energy efficient, increases the yield of methane gas and gives other environmental positive effects at the same time.

Prior art aiming to harvest biomass from the sea has only discussed macroalgae, gathered by culturing or harvest (Boudewijn et al. 1983; Schramm and Lehnberg 1984; Gao and McKinley 1994; Yokoyam et al. 2007; The Crown Estate 2009) as well as macroalgae that has been driven ashore (Detox AB 2009). Even anaerobic digestion of microalgae taken from the algal blooms in the Baltic Sea has been proposed (Gröndahl 2009) as well as anaerobic digestion of blue mussels cultured in the Baltic Sea (Hansson 2008; Radio Kalmar 2009). Also an idea based on culturing microalgae in nutrient rich waste water from sewage plants has been presented (Nyberg 2008; Clear Water Energy Nordic AB 2009). Among filed patent applications several can be mentioned claiming the exclusive right to use biomass based on micro- or macroalgae as well as their extracts (US2008/0050800, CN101418316, US2009081744, EP2014759, CN101285075, CN101255075, UA24106, DE102007007131, WO2007014717, WO9851814, PT100012, DE3607864, CN101418315).

JP 2008 049273 A discloses a treatment method for shellfish where shellfish undergo anaerobic treatment in a treatment tank with anaerobic microorganisms.

JP H08 57462 A discloses a method to separate and treat shell meat and shell of a shellfish by immersing a shellfish in an aqueous solution containing an anaerobic bacteria group to liquefy shell meat as monomeric organic matter and decomposing it to methane gas by methane fermentation bacteria to convert the same to inorganic matter.

JP 2000 033358 A discloses a method for methane fermentation for shellfish which executes the decomposition treatment of shellfish meat and recovers shells as calcium carbonate.

LIM Y.-G. ET AL: "Solubilization and methanogenesis of blue mussels in saline mesophilic anaerobic biodegradation",INTERNATIONAL BIODETERIORATION AND BIODEGRADATION, vol. 61, no. 3, 2007, pages 251-260 discloses biogas production by anaerobic digestion of blue mussels.

KELLY M.S. ET AL: "Marine Estate Research Report - The potential of marine biomass for anaerobic biogas production", 2008, discloses the anaerobic digestion of marine algae and the use of the effluent from digestion as fertilizer.

VERGARA-FERNANDEZ A. ET AL: "Evaluation of marine algae as a source of biogas in a two-stage anaerobic reactor system",BIOMASS AND BIOENERGY, vol. 32, no. 4, April 2008, discloses the anaerobic digestion of marine algae.

US 4 975 106 A discloses a method of anaerobic digestion of fish waste where fish wastes are mixed with a culture of anaerobic microorganisms.

### MARINE BIOMASS- ASCIDIANS

Sea squirts, Ascidiacea, is a class of tunicates with approx 2300 species around the world, of which approximately 50 at the Swedish west coast. They are sessile, solitary or colony forming suspension feeders. The body is sack like and completely surrounded by the tunica. The genus *Ciona* consists of following species: *Ciona edwardsi, Ciona fascicularis, Ciona gelatinosa, Ciona imperfecta, Ciona intestinalis, Ciona mollis* and *Ciona savignyi.* As an example the ascidian *Ciona intestinalis* L. is mentioned which occurs in very high abundances and has been reported in densities over 5000 individuals per m² (Millar 1971). The weight of biomass has been estimated to ~7kg DW/m² (Gulliksen 1972) and in Canadian water to 200kg wet weight / m² (Ramsay, Davidson et al. 2009). If we use the dry matter composition of 4% (Petersen et al. 1997) this value corresponds to 8kg DW/m². They live by filtering plankton from the water and has a very rapid growth compared to many other animals. Their daily growth in length can be 2-3% and a doubling time of weight in 10 days (Petersen et al. 1995). *Ciona intestinalis* reproduces in boreal waters two times per year and in warmer water three times, possibly four times (Dybern 1965).

### Definitions

DW: Abbreviation for Dry Weight which is the water free weight the organism has after drying (∼80° C, 1 day).

Wet weight: The weight of the biomass in wet condition which is weighed within 1 hour after harvest when the biomass is at least half the time in a colander.

Marine / marine environment: Defined as the water environment which is not pure lake water or consists of fresh water. i. e. all water environment which has a salinity over 0.5 ‰ NaCl.

Sea: Same definition as marine environment.

Organism: Defined as all living organisms of the groups Eukaryota, Archea and Procaryote. Animal: A group of organisms belonging to the group Animalia.

Sea squirt / ascidians: Animals belonging to the group Ascidia
Artificial seeding: A by man controlled production of egg and sperms in organisms whose zygotes (the fertilized egg) develops to larvae which settles on hard surfaces in water before they grow to adult animals.

Water: All environments consisting of H2O which includes marine environment and lakes, both environments includes both natural as well as artificial environments, e. g. ponds and other water constructions.

Plankton: All organisms living suspended in water is the scientific correct definition as we use in this text. The plankton can be divided into, and embraces, zooplankton, phytoplankton and bacterioplankton depending on which organism is referred to.

Settla: Is a Swedish translation of the English term settle which is translated to the Swedish *bli fast, satta sig.* The term *settla* (infinitive - *att settla)* is used by Swedish marine biologists.

Ascidia: The organism group is named sjöpungar in Swedish. Ascidia is synonymous with Ascidiacea and is defined by Tree-of-life (http://tolweb.org/tree) and references therein.

Anthozoa: The organism group is named "koraller" in Swedish. Defined by Tree-of-life and references therein.

Porifera: The organism group is named "svampdjur" in Swedish. Defined by Tree-of-life and references therein.

Macroalgae: Visible algae. A unifying term for the organism groups red algae (rhodophyta), green algae (chlorophyta) and brown algae (phaeophyta).

Microalgae: Unicellular organisms which are not visible to the naked eye and are living suspended in the sea. Are not related with macroalgae but belongs to several different organism groups such as dinoflagellates, diatoms etc.

### BRIEF DESCRIPTION OF THE FIGURES

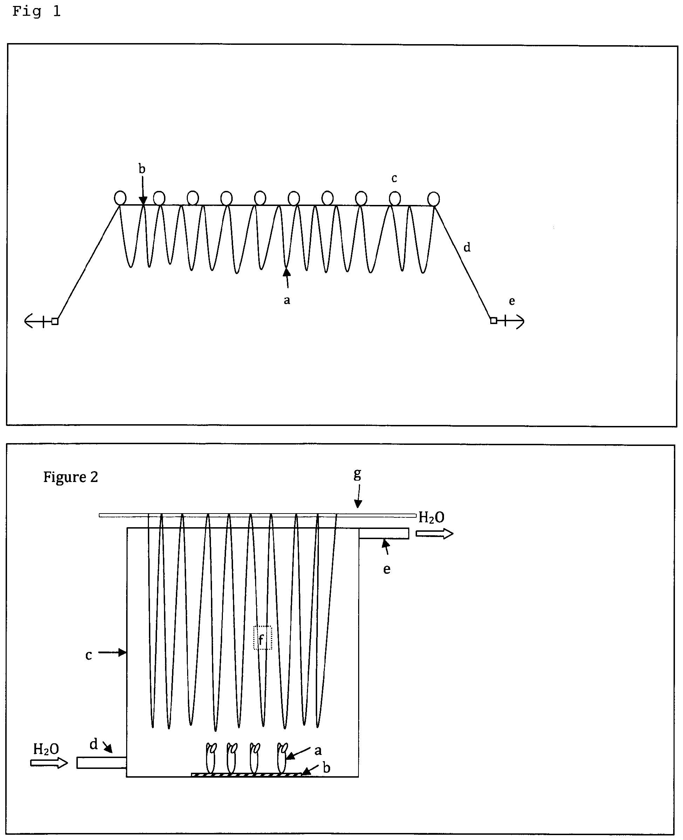

Figure 1: Large scale culturing of ascidians in the sea for biomass production. Ascidians are growing on culture lines (a) which are submersed in the water. A horizontal rope (b) fastened to floats (c) holds the culture lines up. The horizontal lines is trenched outwards by a rope (d) which is hold by moorings and anchors (d).
Figure 2: Description of a method for artificial seeding of ascidian larvae on culture lines submersed in water. Mature individuals of ascidians (a) are fastened on a net (b) inside the water tank (c). Water is entering by a pipe (d) and is exiting by a pipe (e) Culturing lines (f) is hung over a pole (g) which can be removed from the tank.

### SUMMARY

The invention consists of a procedure composed of anaerobic digestion of water living organisms to be used as feedstock for biogas production. The said procedure can also include the culture and harvest of the organisms before anaerobic digestion. A very suitable organism for this is in Swedish waters is the sea squirt organism, *Ciona intestinalis,* based on 1. A very fast growth which allows for several harvests annually, even during winter time 2. No hard internal structures such as shell or bones which prevent the biomass to be pumped 3. The biomass has been shown to increase the biogas production during anaerobic digestion with multiple biomasses 4. Possibility to be cultured in existing culture systems for blue mussels 5. Production ensurement in that it is possible to avoid losses of biomass due to harsh weather which happens to land based agriculture 6. Continuous culturing and harvest season by an, in this patent, described procedure which allows the sea squirts to settle on the culture ropes under controlled conditions which allows for continuous harvest 7. Culturing is without competition to agriculture 8. Culturing and harvest in the sea gives much more biomass per area and demands less energy for culturing and harvest compared to agriculture.

In other countries and waters, other species of ascidians (or corals or sponges) can be most suitable to culture and harvest to achieve the benefits by means of cultivability, rapid growth and the possibility to use industrial pumps in combination with an energy system to produce biogas by anaerobic digestion. Examples on such organisms are *Ascidia sydneiensis,* salps, apendicularians and desmosponges. By using experience and techniques from industrial culturing of mussels this invention secures the industrial production of ascidians. In addition new technical solutions have been incorporated to secure an industrial production of ascidians during the whole year and not only during the ascidians ordinary period of reproduction.

The invention relates to a procedure for biogas production, which comprising anaerobic digestion of water living organisms belonging to the group ascidiacea.

The procedure for biogas production comprises anaerobic digestion and utilization of the produced gas during anaerobic digestion. The digester gas (so called raw gas) consists of methane and carbon dioxide, of this mixture the methane is purified for the production of biogas.

In one embodiment, the procedure includes culture and harvest ascidians before they are anerobically digested.

Culturing of ascidians may be performed by using a number of surfaces submersed in water, on which said surfaces the ascidians can be cultured before harvest.

According to a preferred embodiment, the procedure includes fastening ascidians in larval stage on culturing surfaces, by storing said culturing surfaces submersed in water tanks together with adult individuals of ascidians, regulating the water temperature in the tank, causing the adult individuals to start spawning, and larvae produced by the fusion of gametes, are settling on the surfaces, which are there after placed on the culture site.

Further, the culture can also be achieved according to methods described by Aypa (1990); rack culture, hanging method, bitin culture, tulus culture, stake culture, tray culture, wig-wam culture, rope-web culture, bouchout culture, raft culture and long-line culture.

According to another aspect, the method includes to utilize solid and fluid digestate. The method can further include utilizing the solid and fluid digestate to produce a fertilizer.

The invention also relates to a method for producing fertilizer, comprising anaerobic digestion of water living organisms belonging to the groups ascidians and thereafter utilize solid and fluid digestate for fertilizer production.

Another aspect of the invention relates to a method comprising to culture ascidians together with macroalgae of the groups rhodophyta, chlorophyta and phaeophyta. Combined culture leads to higher growth rate of the macroalgae due to the dissolved nutrients excreted by the ascidian metabolism.

This method can further relate to anaerobically digest the macroalgae together with said ascidians.

The invention also relates to a method for production of fertilizer, comprising culturing and anaerobic digestion of ascidians and macroalgae together, and to utilize solid digestate to produce fertilizer.

In one form of practice the said methods is the organisms filter feeding animals of the groups anthozoa and porifera.

The invention also relates to a method of fastening ascidians in larval stage on culturing surfaces, by storing said culturing surfaces submersed in water tanks together with adult individuals of ascidians, regulating the water temperature in the tank, causing the adult individuals to start spawning, and larvae produced by the fusion of gametes, are settling on the surfaces, which thereafter are placed on the culture site.

Another aspect of the invention comprises anaerobic digested biomass from anaerobic digestion of water living animals from the group Ascidia.

In one embodiment, the invention relates to anaerobic digested biomass from ascidians derived by any of the above mentioned methods.

### DETAILED DESCRIPTION

Below, the present invention will be described in detail with reference to specific embodiments of the invention. This description is provided to increase the understanding of said specific embodiments, and is in no way meant to limit the scope of the present invention. The scope of the present invention is defined by the claims as appended.

### Efficacy as feedstock for anaerobic digestion

The background for the invention to use ascidians as biomass in anaerobic digestion lies in the fact that Swedish producers of biogas are using waste from the seafood industry, in the form of brine and rest products, and so increases the biogas production, both in volume produced biogas as well as a higher percentage of methane in the gas. Waste from the seafood industry is considered to be a very good biomass complement in combined anaerobic digestion with waste sludge, a possible explanation for this could be that the said waste is rich in nitrogen which promotes the overall anaerobic digestion if the combined biomass has a higher composition of carbon. In anaerobic digestion a general rule is that the molar quota between carbon and nitrogen shall be between 30:1 and 20:1 (C:N). Ascidians have a carbon - nitrogen quota of 4:1 which is too low for optimal anaerobic digestion using ascidians alone. But ascidian biomass is very suitable for anaerobic digestion in combination with more carbon rich biomasses such as waste sludge from sewage treatment plants or crops from agriculture. The waste from seafood industry has a carbon - nitrogen quota of 5:1 which is an argument that the low carbon-nitrogen quota of ascidians is not a problem in industrial anaerobic digestion processes.

The salinity of biomass from newly harvested ascidians is more or less the same as the surrounding water. This is caused by the fact the ascidians close their body openings and enclosures a volume of water. In ascidians from the sea outside Lysekil, Sweden, which we have used in tests of anaerobic digestion, the salinity has ranged between 20 and 30 ‰ but can vary between 10 and 35 ‰ in these waters. The waste from seafood industry has a salinity of 60 - 70 ‰ and this salinity has not been shown to decrease the efficacy of anaerobic digestion if the digester is given time to adjust for the amount of saline biomass. The fact that a anaerobic digestion process accepts fair amounts of saline biomass is known (Schramm and Lehnberg 1984; The Crown Estate 2009). It is also known that a small degree of salinity enhance the production of biogas. A general rule is that the bacterial community can handle a wide range of different biomasses given time to adjust. Methane producing bacteria comes evolutionary from the sea but occurs in stomachs of animals as well as in marine and brackish waters.

### Marine biomass - other species of ascidians and corals and sponges

As well as culturing the species we have chosen as an example, *Ciona intestinalis,* which is suitable to culture in Swedish waters, other species of Ciona or other ascidians can be cultured where the environmental conditions so demand or if *Ciona intestinalis* is not naturally occurring. See table 1 for a phylogenetic overview of the class Ascidia.

In the genus *Ciona,* as mentioned above, following species is included: *Ciona edwardsi, Ciona fascicularis, Ciona gelatinosa, Ciona imperfecta, Ciona intestinalis, Ciona mollis* and *Ciona savignyi,* which all is suitable for similar use as *Ciona intestinalis.*

**Table 1 Phylogenetic overview of the class ascidians (Ascidiacea) which all are possible to use as biomass for anaerobic digestion.**

| | | |
|---|---|---|
| Kingdom: | Animalia | |
| Phylum: | Chordata | |
| Subphylum: | Tunicata | |
| Class: | Ascidiacea | |
| Order: | Enteroqona | |
| | Suborder Aplousobranchia | Family Clavelinidae |
| | | Family Didemnidae |
| | | Family Polycitoridae |
| | | Family Polyclinidae |
| | Suborder Phlebobranchia | Family Agnesiidae |
| | | Familj Ascidiidae |
| | | Family Cionidae |
| | | Family Corellidae |
| | | Family Diazonidae |
| | | Family Hypobythiidae |
| | | Family Perophoridae |
| | | Genus Didemnum |
| Order | **Pleurogona** | |
| | Suborder Stolidobranchia | |
| | | Family Pyuridae |
| | | Family Styelidae |
| | | Genus *Eusynstyela* |
| | | Genus *Styela* |
| | | Family Botryllidae |
| | | Genus *Botrylloides* |
| | | Genus *Botryllus* |

Other fast growing species of both corals and sponges can also be cultured in the sea, or in lakes, with the same methods as described herein, aiming at producing biogas for anaerobic digestion or production of bioethanol. Some of these animals can be suitable for culturing off-shore at larger depths. In expanding the examples given here, we point at the high abundance and productivity of animals around so called "thermal vents" (hot underwater volcanoes) where many life forms is using chemotrophic bacteria as primary producers. In this patent application we have chosen to use ascidians as examples. The invention is thereby not limited to the use of ascidians, but includes coral and sponge animals which neither is industrial cultured to producing biomass.

### Culturing of organisms in water

A general system for culturing sessile organisms in sea, either marine or brackish, is that it consists of a surface submersed in water where organisms from the ambient water settle as larvae or that larvae or spores settles on the surfaces in water tanks on land by manipulating the parental generation. Generally at harvest the surfaces, as ropes, lines, poles, nets, etc., is removed from the water so the organisms can be harvested onto a boat or to land. Another occurring method is to culture organisms on natural substrates and bottoms which also give the possibility for production of biomass.

To culture ascidians, corals or sponges, the general system for culturing mentioned above, is sufficient if the submersed surface has the right properties for the organisms to settle and grow and not to fall of the surface. The suitable time for submersion of the surfaces is important regarding water temperature, salinity or other physical parameters to ensure a good growth of the animals. The time for submersion is also important regarding the presence of larvae in the water due to their life cycle. Below are a number of culture methods given as examples for methods suitable for ascidian culture which also is suitable for culturing of corals and sponges. Several of these methods are given in aqua culture literature (see e.g. Aypa 1990).

The long-line method is most commonly used for mussel culturing in Sweden and the method as we find, in Swedish waters, as the most suitable. This is based on that the method is used in industrial scale for culture and harvest of approx. 3000 tons blue mussels annually and the methods is therefore reliable. All equipment is commercial available and harvest boats is possible to rent as well as experienced personnel. The long-line method consists of a polypropylene band, 5 cm width, which is submersed into water in a long unbroken chain. In a Swedish long-line culture with dimensions 20*200 meters the total length of culture lines is 24 km. The lines is fastened with regular intervals on a carrier rope which in turn is uphold by floats, see Figure 1. The ends of the carrier ropes are anchored to keep the culture outstretched. The method is also named "one dragon method" in China. By placing the culture lines hanging above the bottom, bottom living animals is efficiently hindered to come up onto the lines and feed, for *Ciona intestinalis* this is valid for e.g. *Asterias rubens* which feeds on the said species.

Other methods that can be used for efficient culture and harvest can include (according to Aypa 1990): Rack culture, hanging method, bitin culture, tulus culture, stake culture, tray culture, wig-wam culture, rope-web culture, bouchout culture, raft culture, long-line culture.

### Integrated culture

A specific embodiment of the method to culture ascidians in the sea with the purpose to produce biomass for anaerobic digestion is to have an integrated culture of ascidians and macro algae. Different techniques for culturing macro algae are well known and are described well in the specialist literature (Lucas and Southgate 2003; Pillay and Kutty 2005). It is also known that macroalgae have a higher growth rate when there are higher concentrations of nutrients in the water, mostly nitrogen and phosphorus compounds. It is also known that animals excrete nutrients as byproducts of their metabolism in the form of e.g. urine, urea or substances with similar purpose to remove excess metabolites from organisms. These excretions are rich in easy available nitrogenous substances; compare e.g. the use of manure as fertilizer in agriculture. With this background knowledge we state that there is an enhancing effect in integrated culture of ascidians, or the organisms chosen for the production of biomass for anaerobic digestion, with macro algae. The ascidians are excreting nutrients that make macroalgae grow faster which is preferable for industrial producers. In an environmental perspective, said integrated culture increases the capacity to remove nutrients from the sea for the whole cultures which is positive if emission credits is sold based on the facto amount of nitrogen and phosphorus that is removed from the sea. No one has previously used an integrated culture of ascidians with macroalgae as biomass for anaerobic digestion to achieve the said effect.

The integrated culture is obtained by the following description: 1. Ascidians and macroalgae is cultured on different rope/band within the same culture unit so the macroalgae comes in proximity to the release of nutrients from the ascidians and the ascidians does not compete for planktonic food 2. Ascidians and macroalgae is cultured on separate but closely located units.

The maximum distance between culturing units, when culturing separately, is determined by the size of the culturing units as well as water currents and hydrography of the area so the macroalgae can utilize the nutrients that the ascidians excrete.

### Artificial seeding

*Ciona intestinalis* is reproducing in temperatures between 8° and 23° (Dybern 1965) and releases larvae in Swedish and Norwegian waters between May and September with maximum larval production in June (Dybern 1965; Gulliksen 1972).

The method of artificial seeding is today used for cultivation of macroalgae such as species of *Porhyra* among which one is the popular Japanese Nori-algae used for sushi (Kelly and Dworjanyn 2008). Artificial seeding means that by performing a certain technical procedure, such as manipulating the amount, quality and temperature of the light, the lifecycle of the macroalgae can be regulated to produce reproductive bodies (spores). For a person skilled in the art it is possible to perform such a method.

However, no one has used the mentioned method to ensure settling on culture surfaces in the sea for ascidians. Knowledge of the physical properties of the water that is needed for ascidians to release their egg and sperms in laboratory is well known. This knowledge has been used to rear new individuals of ascidians for research purposes. But since no one has had the idea to culture ascidians in large scale at sea, no one has had the idea to improve such method by artificial seeding.

The method is performed by storing adult individuals of ascidians in a water tank where the water temperature is kept below 8°, at which temperature ascidians get sexually mature if the temperature is exceeded. In those tanks the culture band, culture ropes or other culture surfaces, onto which the ascidians shall settle as a new generation larvae, are submersed. Thereafter the temperature is raised over 8° and the adult individuals of ascidians after a period of time releases egg and sperm which is conjugated to larvae. The larvae will thereafter settle on the submersed culture band, culture ropes or other culture surfaces.

The ascidians can be contained in the same tank as the culture bands or be contained in a tank with communicating water connection.

After the settling of larvae on the submersed band the larvae are growing for a suitable delimited time until they have had an enough growth to stand transportation to the new water environment where they shall grow to adult age before harvest.

### Results

The growth rate of *Ciona intestinalis* has been measured by the applicant and was found to be 8.5 cm (n=350, s.d. = 3.2 cm) during 93 days and a increase of biomass, measured as wet weight per m², of 33 kg during the same period of time (location: Lysekil, Sweden, time May 2009 to August 2009, depth 1-2 meter on a vertical concrete construction, approximate salinity 20-34 psu, approximate temperature 10-20°).

The inventor has further conducted anaerobic digestion tests where the production of biogas after 10 days was 600 ml CH₄/g VS with biomass from ascidia mixed with 30 % (weight) carbon rich biomass.

The inventor has also conducted test for harvest of ascidians (*Ciona intestinalis)* on an ascidian culture in Ljungskile on the Swedish west coast during April 2010. The culture was using the long-lines method. During the harvest test a boat ordinary used for blue mussel harvest did harvest 10m³ ascidians per hour.

The inventor has conducted a test for anaerobic digestion of 10m³ ascidians during April 2010. The ascidian biomass was digested in combination with sludge waste from a waste water plant. The results did show that the biogas production after mixing with 5% Ciona intestinalis was retained at previous levels as before the addition. The concentration of methane was unchanged and varied between 58 and 64%.

### REFERENCES

Radio Kalmar (2009). Blåmusslor ska rädda Östersjön. Radio P4 - Kalmar. Kalmar, Sweden.
Aypa, M. S. (1990). Selected papers on mollusc culture. Regional Seafarming Development and Demonstration Project. Project reports - SF/WP/90/2. FAO, FAO (Food and Agricultural Organization).
Boudewijn, H. B., A. B. Valeria, et al. (1983). "NEW YORK MARINE BIOMASS PROGRAM: CULTURE OF Laminaria saccharina." Journal of the World Mariculture Society 14(1-4): 360-379.
Clear Water Energy Nordic AB. (2009). "Resurs i vatten med näring." Retrieved 2009-08-25, 2009, from http://www.cwenordic.se/index.htm].
Detox AB. (2009). "Exempel på projekt - Biogas." Retrieved 2009-08-25, 2009, from http://www.detox.se/.
Dybern, B. I. (1965). "The life cycle of Ciona intestinalis (L.) f. typica in relation to the environmental temperature." Oikos 16:109-131.
Gao, K. and K. R. McKinley (1994). "Use of macroalgae for marine biomass production and CO2 remediation: a review." Journal of Applied Phycology 6: 45-60.
Gröndahl, F. (2009). Sustainable use of Baltic Sea natural resources based on ecological engineering and biogas production. Interdisciplinary Workshop: Effects of climate change: coastal systems, policy implications, and the role of statistics. Sliema, Malta.
Gulliksen, B. (1972). "Spawning, larval settlement, growth, biomass and distribution of Ciona intestinalis L. (tunicata) in Borgensfjorden, north-Tröndelag, Norway " Sarsia 51: 83-96.
Hansson, L.-A. (2008). Kan Östersjön restaureras? Utvärdering av erfarenheter från sjöarna. Rapport 5860. Naturvårdsverket, Swedish Environmental Protection Agency.
Kelly, M. S. and S. Dworjanyn (2008). The potential of marine biomass for anaerobic biogas production: a feasibility study with recommendations for further research, The Crown Estate on behalf of the Marine Estate.
Lucas, J. S. and P. C. Southgate, Eds. (2003). Aquaculture. Farming Aquatic Animals and Plants Fishing News Books.
Millar, R. H. (1971). "The biology of ascidians." Adv. mar. Biol. 9:1-100.
Nyberg, M. (2008). "Så ska de rädda Ostersjon och tjäna miljoner." Retrieved 2009-08-25, 2009, from http://miljoaktuellt.idg.se/2.1845/1.192579/sa-ska-de-radda-ostersjon-och-tjana-miljoner.
Petersen, J. K., O. Shou, et al. (1995). "Growth and energetics in the ascidian Ciona intestinalis." Marine Ecology Progress Series 120:175-184.
Petersen, J. K., O. Shou, et al. (1997). "IN situ growth of the ascidian Ciona intestinalis (L.) and the blue mussel Mytilus edulis in an eelgrass meadow." Journal of Experimental Marine Biology and Ecology 218:1-11.
Pillay, T. V. R. and M. N. Kutty (2005). Aquaculture. Principles and Practices, 2nd ed. Blackwell Publishing Ltd.
Ramsay, A., J. Davidson, et al. (2009). "Recruitment patterns and population development of the invasive ascidian Ciona intestinalis in Prince Edward Island, Canada." Aquatic Invasions 4(1):169-176.
Schramm, W. and W. Lehnberg (1984). "Mass culture of brackish-water-adapted seaweeds in sewage-enriched seawater. II: Fermentation for biogas production." Hydrobiologia 116/117: 282-287.
The Crown Estate. (2009). "Aquaculture research." Retrieved 2009-08-25, 2009, from http://www.thecrownestate.co.uk/mrf aquaculture.
Tree of life. (2009). "Tree of life web project." Retrieved 2009-08-25, 2009, from http://tolweb.org/tree/.
Yokoyama, S., K. Jonouchi, et al. (2007). "Energy Production from Marine Biomass: Fuel Cell Power Generation Driven by Methane Produced from Seaweed." World Academy of Science. Engineering and Technology 28: 320-323.

## Claims

1. A method for production of biogas, comprising anaerobic digestion of water living organisms of the species Ciona intestinalis and Ciona savignyi in combination with more carbon rich biomass.

2. The method according to claim 1, further comprising culturing and harvesting the species Ciona intestinalis and Ciona savignyi before anaerobic digestion.

3. The method according to claim 2, comprising culture by using a number of surfaces which are submersed in water, on which surfaces the organisms are cultured before harvest.

4. The method according to claim 2 or 3, comprising fastening the species Ciona intestinalis and Ciona savignyi in larval stage on culturing surfaces, by storing said surfaces submersed in water tanks together with adult individuals of the species Ciona intestinalis and Ciona savignyi, to control the water temperature in the tank, causing the adult individuals to start spawning, and larvae that are produced by fusion of gametes, are allowed to settle on the submersed surfaces, which thereafter are transported to the culture site.

5. The method according to any of the claims 2-4, in which the culture is performed by using any of the following methods; rack culture, hanging method, bitin culture, tulus culture, stake culture, tray culture, wig-wam culture, rope-web culture, bouchout culture, raft culture and long-line culture.

6. The method according to any of the preceding claims, comprising utilizing solid and fluid digestates.

7. The method according to claim 6, comprising utilizing the solid and fluid digestate for production of fertilizer.

8. A method for production of fertilizer, comprising anaerobic digestion of water living animals of the species Ciona intestinalis and Ciona savignyi and utilizing solid and fluid digestate for production of fertilizer.

9. The method according to any of the claims 2-5, comprising an integrated culture of the species Ciona intestinalis and Ciona savignyi and macroalgae from any of the groups rhodophyta, chlorophyta and phaeophyta.

10. The method according to claim 9, comprising anaerobic digestion of the macroalgae together with the species Ciona intestinalis and Ciona savignyi.

11. The method according to claim 10, further comprising the utilization of solid and fluid digestate.

12. The method according to claim 11, further comprising using the solid and fluid digestate to produce fertilizer.

13. The method according to claim 8, further comprising culturing and anaerobically digesting the species Ciona intestinalis and Ciona savignyi and macroalgae together, and utilizing the solid and fluid digestate for production of fertilizer.

14. Anaerobic digested biomass obtained by anaerobic digestion of water living organisms from the species Ciona intestinalis and Ciona savignyi.

## Patentansprüche

1. Verfahren zur Herstellung von Biogas, umfassend die anaerobe Digestion von in Wasser lebenden Organismen der Arten Ciona intestinalis und Ciona savignyi in Kombination mit weiterer kohlenstoffreicher Biomasse.

2. Verfahren nach Anspruch 1, weiters umfassend die Kultivierung und Ernte der Arten Ciona intestinalis und Ciona savignyi vor der anaeroben Digestion.

3. Verfahren nach Anspruch 2, umfassend die Kultivierung durch Verwendung einer Anzahl von in Wasser untergetauchten Oberflächen, auf welchen Oberflächen die Organismen vor der Ernte kultiviert werden.

4. Verfahren nach Anspruch 2 oder 3, umfassend das Festsetzen der Arten Ciona intestinalis und Ciona savignyi im Larvenstadium auf zur Kultivierung geeigneten Oberflächen durch Lagerung der in Wassertanks untergetauchten Oberflächen zusammen mit erwachsenen Individuen der Arten Ciona intestinalis und Ciona savignyi, die Kontrolle der Wassertemperatur im Tank, das Veranlassen der erwachsenen Individuen mit der Vermehrung zu beginnen und das Absetzen lassen von durch die Fusion von Gameten produzierten Larven auf die untergetauchten Oberfläche, welche danach zur Kultivierungsstelle transportiert werden.

5. Verfahren nach einem der Ansprüche 2 bis 4, worin die Kultivierung durch Verwendung irgend einer der folgenden Methoden durchgeführt wird; Rahmenkultur, hängende Methode, Bitinkultur, Tuluskultur, Pfahlkultur, Trogkultur, Wigwamkultur, Seil-Netzkultur, Bouchoutkultur, Floßkuttur und Langleinenkultur.

6. Verfahren nach einem der vorhergehenden Ansprüche, umfassend die Verwendung von festen und flüssigen Digestaten.

7. Verfahren nach Anspruch 6, umfassend die Verwendung der festen und flüssigen Digestaten für die Herstellung von Düngemittel.

8. Verfahren zur Herstellung von Düngemittel, umfassend die anaerobe Digestion von im Wasser lebenden Tieren der Arten Ciona intestinalis und Ciona savignyi und die Verwendung von festen und flüssigen Digestaten zur Herstellung von Düngemittel.

9. Verfahren nach einem der Ansprüche 2 bis 5, umfassend eine integrierte Kultur der Arten Ciona intestinalis und Ciona savignyi und Makroalgen aus einer der Gruppen Rhodophyta, Chlorophyta und Phaeophyta.

10. Verfahren nach Anspruch 9, umfassend die anaerobe Digestion der Makroalgen zusammen mit den Arten Ciona intestinalis und Ciona savignyi.

11. Verfahren nach Anspruch 10, weiters umfassend die Verwendung von festen und flüssigen Digestaten.

12. Verfahren nach Anspruch 11, weiters umfassend das Verwenden der festen und flüssigen Digestaten für die Herstellung von Düngemittel.

13. Verfahren gemäß Anspruch 8, weiters umfassend die gemeinsame Kultivierung und anaerobe Digestion der Arten Ciona intestinalis und Ciona savignyi und Makroalgen und die Verwendung der festen und flüssigen Digestaten für die Herstellung von Düngemittel.

14. Anaerobe verdaute Biomasse erhalten durch anaerobe Digestion von in Wasser lebenden Organismen aus den Arten Ciona intestinalis und Ciona savignyi.

## Revendications

1. Procédé de production de biogaz, comprenant la digestion anaérobie d'organismes vivants dans l'eau de l'espèce Ciona intestinalis et Ciona savignyi en combinaison avec une biomasse plus riche en carbone.

2. Procédé selon la revendication 1, comprenant en outre la culture et la récolte de l'espèce Ciona intestinalis et Ciona savignyi avant la digestion anaérobie.

3. Procédé selon la revendication 2, comprenant une culture en utilisant un nombre de surfaces qui sont immergées dans l'eau, sur lesquelles surfaces les organismes sont cultivés avant la récolte.

4. Procédé selon la revendication 2 ou 3, comprenant la fixation de l'espèce Ciona intestinalis et Ciona savignyi à l'état larvaire sur des surfaces de culture, en stockant lesdites surfaces immergées dans des réservoirs d'eau conjointement avec des individus adultes de l'espèce Ciona intestinalis et Ciona savignyi, afin de commander la température de l'eau dans le réservoir, pour amener les individus adultes à commencer la fraie, et des larves qui sont produites par la fusion de gamètes, sont autorisées à se déposer sur les surfaces immergées qui ensuite sont transportées sur le site de culture.

5. Procédé selon une quelconque des revendications 2 à 4, dans lequel la culture est réalisée en utilisant un quelconque des procédés suivants : culture sur râtelier, procédé de suspension, culture bitin, culture tulus, culture sur poteaux, culture sur plateaux, culture wig-wam, culture sur âme câblée, culture de bouchout, culture sur radeaux et culture sur ligne longue.

6. Procédé selon une quelconque des revendications précédentes, comprenant l'utilisation de produits de digestion solides et fluides.

7. Procédé selon la revendication 6, comprenant l'utilisation de produits de digestion solides et fluides pour la production d'engrais.

8. Procédé de production d'engrais, comprenant une digestion anaérobie d'animaux vivants dans l'eau de l'espèce Ciona intestinalis et Ciona savignyi et l'utilisation de produits de digestion solides et fluides pour la production d'engrais.

9. Procédé selon une quelconque des revendications 2 à 5, comprenant une culture intégrée de l'espèce Ciona intestinalis et Ciona savignyi et de macroalgues provenant des groupes rhodophyta, chlorophyta et phaeophyta.

10. Procédé selon la revendication 9, comprenant une digestion anaérobie des macroalgues conjointement avec l'espèce Ciona intestinalis et Ciona savignyi.

11. Procédé selon la revendication 10, comprenant en outre l'utilisation de produits de digestion solides et fluides.

12. Procédé selon la revendication 11, comprenant en outre l'utilisation des produits de digestion solides et fluides pour produire des engrais.

13. Procédé selon la revendication 8, comprenant en outre la culture et la digestion de façon anaérobie de l'espèce Ciona intestinalis et Ciona savignyi et de macroalgues ensembles, et l'utilisation de produits de digestion solides et fluides pour la production d'engrais.

14. Biomasse digérée de façon anaérobie obtenue par une digestion anaérobie d'organismes vivants dans l'eau provenant de l'espèce Ciona intestinalis et Ciona savignyi.
